# EUROPEAN PATENT SPECIFICATION

(11) **EP 4 092 025 B1**
(45) Date of publication and mention of the grant of the patent: **14.08.2024**
(21) Application number: 21741236.0
(22) Date of filing: 15.01.2021
(51) Int. Cl.: A61K 9/00, A61K 9/10, C07D 471/04, A61K 31/519, A61K 31/517, A61K 9/72, A61P 11/08, A61K 47/26

(54) **PHARMACEUTICAL COMPOSITION OF TRICYCLIC PDE3/PDE4 DUAL INHIBITOR COMPOUND**
PHARMAZEUTISCHE ZUSAMMENSETZUNG EINER TRICYCLISCHEN PDE3/PDE4-DOPPELINHIBITORVERBINDUNG
COMPOSITION PHARMACEUTIQUE DE COMPOSÉ INHIBITEUR DOUBLE DE PDE3/PDE4 TRICYCLIQUE

(30) Priority: 15.01.2020 CN 202010042865
(43) Date of publication of application: 23.11.2022
(73) Proprietor: CHIA TAI TIANQING PHARMACEUTICAL GROUP CO., LTD., Lianyungang, Jiangsu 222062 (CN)
(72) Inventor: YANG, Miao, Lianyungang, Jiangsu 222062 (CN); SUN, Yuanyuan, Lianyungang, Jiangsu 222062 (CN); LI, Wanjing, Lianyungang, Jiangsu 222062 (CN); XU, Zhong, Lianyungang, Jiangsu 222062 (CN); BAO, Pengyue, Lianyungang, Jiangsu 222062 (CN); GAO, Jing, Lianyungang, Jiangsu 222062 (CN); DONG, Ping, Lianyungang, Jiangsu 222062 (CN); HUI, Hui, Lianyungang, Jiangsu 222062 (CN); JI, Xiongfeng, Lianyungang, Jiangsu 222062 (CN); LUO, Yunfu, Shanghai 200131 (CN)
(74) Representative: Müller Schupfner & Partner Patent- und Rechtsanwaltspartnerschaft mbB (Muc)
(86) International application number: PCT/CN2021/072145
(87) International publication number: WO 2021/143841

(56) References cited:
- WO-A1-2016/042313
- WO-A1-2020/011254
- WO-A1-2020/011254
- WO-A1-2020/011254
- CN-A- 1 348 453
- CN-A- 103 313 985
- CN-A- 106 794 157
- US-A- 4 482 556
- US-A- 4 581 172

## Description

### CROSS-REFERENCE TO RELATED APPLICATIONS

The present application claims the benefit and priority to the Chinese Patent Application No. 202010042865.X filed to China National Intellectual Property Administration on Jan. 15, 2020, which is incorporated herein by reference in its entirety.

### TECHNICAL FIELD

The present application relates to the technical field of pharmaceutics, specifically to a pharmaceutical composition of a tricyclic PDE3/PDE4 dual inhibitor compound, a method for preparing the same and use thereof, and more specifically to a pharmaceutical composition of a compound of formula (I) or a pharmaceutically acceptable salt thereof, a method for preparing the same and use thereof.

### BACKGROUND

Phosphodiesterases (PDEs) are a superfamily of enzyme systems including 11 members that participate in different signaling pathways and regulate different physiological processes. Among them, PDE3 is a major phosphodiesterase in human airway smooth muscle (ASM), and inhibition of PDE3 increases intracellular cAMP concentration and thus slackens bronchial smooth muscle. PDE4 plays a major regulatory role in the expression of proinflammatory and anti-inflammatory mediators, and a PDE4 inhibitor can inhibit the release of harmful mediators from inflammatory cells. Thus, in theory, an inhibitor that inhibits both PDE3 and PDE4 would have both the bronchodilation of a beta-adrenoreceptor agonist and the anti-inflammatory action of an inhaled glucocorticoid. The functional complementation of dual targeting is theoretically more effective than a sole targeting, providing a therapeutic effect which can be achieved only by a combination at present is achieved through a monotherapy and thus eliminating the defect that the physicochemical properties of the ingredients of medicaments used in a combination cannot be completely matched. In this way, the administration is simplified, and is convenient for a fixed dose regimen.

Victoria Boswell et al, J. Pharmaco. Experi. Therap., 2006, 318:840-848 and WO200005830 reported that compounds RPL554 and RPL565 have long-acting bronchodilator and anti-inflammatory effect, as well as poor solubility, high plasma clearance and other physicochemical properties, and are suitable for inhalational administration. But the data also showed that the PDE4 inhibitory activity is unsatisfactory, and the anti-inflammatory effect is insufficient. Thus there's still a need for developing a compound having good PDE3/4 inhibitory activity.

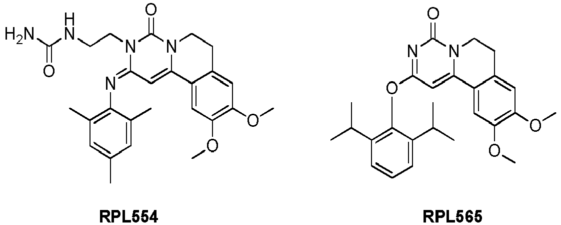

### SUMMARY

In one aspect, the present application provides a pharmaceutical composition comprising a compound of formula (I) or a pharmaceutically acceptable salt thereof, and a surfactant,

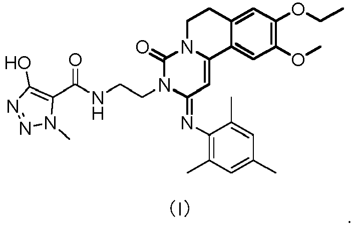

In some embodiments, the pharmaceutical composition further comprises a metal chelating agent (or metal complexing agent).

In some embodiments, the pharmaceutical composition further comprises a buffering agent.

In some embodiments, the pharmaceutical composition further comprises a diluent.

In some embodiments, the pharmaceutical composition further comprises an osmotic pressure regulator.

In some embodiments, the pharmaceutical composition comprises the compound of formula (I) or the pharmaceutically acceptable salt thereof, the surfactant and the buffering agent.

In some embodiments, the pharmaceutical composition comprises the compound of formula (I) or the pharmaceutically acceptable salt thereof, the surfactant, the buffering agent and the osmotic pressure regulator.

In some embodiments, the pharmaceutical composition comprises the compound of formula (I) or the pharmaceutically acceptable salt thereof, the surfactant, the buffering agent and the metal chelating agent.

In some embodiments, the pharmaceutical composition comprises the compound of formula (I) or the pharmaceutically acceptable salt thereof, the surfactant, the buffering agent, the osmotic pressure regulator and the metal chelating agent.

In some embodiments, the pharmaceutical composition comprises the compound of formula (I) or the pharmaceutically acceptable salt thereof, the surfactant, the buffering agent, the osmotic pressure regulator, the metal chelating agent and the diluent.

In some embodiments, the pharmaceutical composition comprises the compound of formula (I) or the pharmaceutically acceptable salt thereof, the surfactant, and at least one of the buffering agent, the osmotic pressure regulator, the metal chelating agent and the diluent.

In some embodiments, the pharmaceutically acceptable salt is selected from the group consisting of maleate, sulfate, methanesulfonate, or *p*-toluenesulfonate.

In some embodiments, in the pharmaceutically acceptable salt of the compound of formula (I), the molar ratio of the compound of formula (I) to the acid radical ion forming the pharmaceutically acceptable salt can be 1:1 to 1:2, e.g., 1:1 or 1:2.

In some embodiments, in the pharmaceutical composition, "the compound of formula (I) or the pharmaceutically acceptable salt thereof" can be used interchangeably with "the compound of formula (I)".

In some embodiments, the pharmaceutical composition comprises the compound of formula (I), the surfactant, the buffering agent, the osmotic pressure regulator, the metal chelating agent and the diluent.

In some embodiments, in the pharmaceutical composition, the compound of formula (I) or the pharmaceutically acceptable salt thereof, based on the compound of formula (I), has a concentration of about 0.001 mg/mL to about 80 mg/mL, preferably 0.002 mg/mL to 50 mg/mL, and more preferably 0.1 mg/mL to 20 mg/mL.

The surfactant of the present application is a pharmaceutically acceptable surfactant, e.g., a wetting agent. The surfactant can be a non-ionic surfactant, an anionic surfactant, a cationic surfactant, or a zwitterionic surfactant. Preferably, one or more of the surfactants are non-ionic surfactants.

In some embodiments, the surfactant is one or more selected from the group consisting of polyoxyethylene glycol, polypropylene glycol alkyl ether, alkyl polyglucoside, octylphenol polyoxyethylene ether, alkylphenol polyoxyethylene ether, glycerin alkyl ester, polyoxyethylene sorbitan fatty acid ester (polysorbate), sorbitan alkyl ester, sorbitan fatty acid ester, cocamide MEA, cocamide DEA, dodecyldimethylamine oxide, a block copolymer of polyethylene glycol and polypropylene glycol (poloxamer), and polyethoxylated tallow amine (POEA). Preferably, the surfactant is one or more selected from the group consisting of polyoxyethylene sorbitan fatty acid ester (e.g., a Tween) and sorbitan fatty acid ester (e.g., a Span).

In some embodiments, the polyoxyethylene sorbitan fatty acid ester is one or more selected from the group consisting of polysorbate 20 (polyoxyethylene sorbitan laurate; Tween 20), polysorbate 40 (polyoxyethylene sorbitan monopalmitate), polysorbate 60 (polyoxyethylene sorbitan stearate) and polysorbate 80 (polyoxyethylene sorbitan monooleate; Tween 80).

In some embodiments, the sorbitan fatty acid ester is one or more selected from the group consisting of sorbitan monolaurate (Span 20), sorbitan monopalmitate, sorbitan monostearate, sorbitan tristearate and sorbitan monooleate.

More preferably, the surfactant is one or more selected from the group consisting of a Tween and a Span.

In some specific embodiments, the surfactant is one or more selected from the group consisting of Tween 20, Tween 80 and Span 20.

In some embodiments, the surfactant has a concentration of about 0.01 mg/mL to about 8 mg/mL. More typically, the concentration of the surfactant in the pharmaceutical composition is about 0.01 mg/mL to 5 mg/mL, preferably about 0.02 mg/mL to 3 mg/mL, more preferably about 0.05 mg/mL to 2 mg/mL, and even more preferably about 0.1 mg/mL to 1 mg/mL.

In some embodiments, in the pharmaceutical composition, the mass ratio of the compound of formula (I) or the pharmaceutically acceptable salt thereof (based on the compound of formula (I)) to the surfactant is about 1:200 to 100:1, preferably about 1:150 to 50:1, more preferably about 1:50 to 25:1, and even more preferably about 1:1 to 15:1, for example, about 10:1.

In some embodiments, the buffering agent is a pharmaceutically acceptable buffering agent. The buffering agent can be any buffer suitable for use in a liquid pharmaceutical composition suitable for inhalation. The buffering agent is generally one or more selected from the group consisting of sulfuric acid, hydrochloric acid, sodium hydroxide, citric acid, sodium citrate, lactic acid, sodium lactate, acetic acid, sodium acetate, trisodium phosphate, sodium dihydrogen phosphate, disodium hydrogen phosphate, potassium dihydrogen phosphate, tartaric acid, sodium tartrate, glycine, boric acid and phthalic acid. The preferred number of the buffering agent is 2 or more than 2, and the preferred type of the buffering agent is citrate buffered saline or phosphate buffered saline, more preferably the sodium salt of citric acid or phosphoric acid. The citrate buffered saline includes citric acid, sodium citrate, and a mixture thereof. The phosphate buffered saline includes phosphoric acid, monosodium phosphate (i.e., sodium dihydrogen phosphate), disodium hydrogen phosphate, and a mixture thereof.

In some embodiments, the buffering agent is selected from the group consisting of citric acid, a citrate (e.g., sodium citrate), tartaric acid, a tartrate (e.g., sodium tartrate), phosphoric acid and a phosphate (e.g., sodium dihydrogen phosphate and disodium hydrogen phosphate).

In some embodiments, the buffering agent is selected from the group consisting of a citrate (e.g., sodium citrate), a tartrate (e.g., sodium tartrate) and a phosphate (e.g., sodium dihydrogen phosphate and disodium hydrogen phosphate).

In some embodiments, the buffering agent has a concentration of about 0.01 mg/mL to about 50 mg/mL, preferably about 0.05 mg/mL to about 40 mg/mL, more preferably 0.1 mg/mL to about 25 mg/mL, and even more preferably 0.5 mg/mL to about 6 mg/mL.

In some embodiments, the buffering agent is used to control the pharmaceutical composition at pH between about 3.0 and about 8.5, preferably between about 5 and about 7.

In some embodiments, the osmotic pressure regulator is generally one or more selected from the group consisting of a simple non-toxic salt, such as sodium chloride, potassium chloride and the like, or a saccharide, such as one or more of glucose, mannitol or xylitol and the like. In some embodiments, the osmotic pressure regulator is sodium chloride.

The concentration of the osmotic pressure regulator depends on the amount needed to achieve a desired isotonicity, e.g., isotonic to plasma or lung fluid. The concentration of the osmotic pressure regulator is typically about 0.01 mg/mL to about 10 mg/mL, and more typically about 5 mg/mL to 9 mg/mL.

In some embodiments, the metal chelating agent is one or more selected from the group consisting of edetic acid and an edetate such as disodium edetate, calcium disodium edetate, and the like. Edetates (e.g., calcium salts, sodium salts) are preferred, and disodium edetate (EDTA-2Na) is particularly preferred.

The concentration of the metal chelating agent depends on the amount of metal ions that can be introduced during the preparation of the pharmaceutical composition and is generally about 0.01 mg/mL to about 40 mg/mL, preferably about 0.01 mg/mL to about 20 mg/mL, more preferably about 0.01 mg/mL to about 5 mg/mL, and even more preferably about 0.01 mg/mL to about 2 mg/mL.

In some embodiments, in the pharmaceutical composition, the diluent can be any pharmaceutically acceptable diluent. The diluent is suitable for inhalational administration. Generally, the diluent is one or more selected from the group consisting of water, ethanol and glycerol. A preferred diluent is water, and a more preferred diluent is sterile water.

In some embodiments, in the pharmaceutical composition, the amount of the diluent used can be an appropriate amount, such that the concentration of the compound of formula (I) or the pharmaceutically acceptable salt thereof or an excipient in the pharmaceutical composition is within a certain range.

In some embodiments, the pharmaceutical composition comprises the compound of formula (I), and the Tween or the Span; furthermore, the Tween or the Span is one or more selected from the group consisting of Tween 20, Tween 80, and Span 20.

In some embodiments, the pharmaceutical composition further comprises the phosphate; furthermore, the phosphate can be selected from the group consisting of sodium dihydrogen phosphate or monohydrate thereof, and disodium hydrogen phosphate; in some embodiments, the pharmaceutical composition further comprises sodium citrate or sodium tartrate.

In some embodiments, the pharmaceutical composition comprises the compound of formula (I), a mixture of the Span and the Tween (such as Tween 80, Tween 20 or Span 20), and water.

In some embodiments, the pharmaceutical composition comprises the compound of formula (I), the mixture of the Span and the Tween (such as Tween 80, Tween 20 or Span 20), sodium citrate or sodium tartrate, and water.

In some embodiments, the pharmaceutical composition comprises the compound of formula (I), the Tween (such as Tween 80 or Tween 20), sodium dihydrogen phosphate or monohydrate thereof, disodium hydrogen phosphate, disodium edetate and water.

In some embodiments, the pharmaceutical composition comprises the compound of formula (I), the Tween (such as Tween 80 or Tween 20), sodium dihydrogen phosphate or monohydrate thereof, disodium hydrogen phosphate, sodium chloride, disodium edetate and water.

In some embodiments, the pharmaceutical composition comprises the compound of formula (I), the Tween (such as Tween 80 or Tween 20), sodium citrate or sodium tartrate, sodium chloride, and water.

In some embodiments, the pharmaceutical composition comprises the compound of formula (I), the mixture of the Span and the Tween (such as Tween 80, Tween 20 or Span 20), sodium citrate or sodium tartrate, sodium chloride and water.

In some embodiments, the pharmaceutical composition comprises the compound of formula (I), the surfactant, the buffering agent, the osmotic pressure regulator, the metal chelating agent and the diluent, wherein the compound of formula (I) has a concentration of 0.002 mg/mL to 50 mg/mL, the surfactant has a concentration of 0.02 mg/mL to 3 mg/mL, the buffering agent has a concentration of 0.1 mg/mL to about 25 mg/mL, the osmotic pressure regulator has a concentration of 5 mg/mL to 9 mg/mL, and the metal chelating agent has a concentration of 0.01 mg/mL to about 5 mg/mL.

In some embodiments, in the pharmaceutical composition, the surfactant, the buffering agent, osmotic pressure regulator, the metal chelating agent and the diluent are as defined above.

In some embodiments, the pharmaceutical composition comprises the compound of formula (I), the surfactant, the buffering agent, the osmotic pressure regulator, the metal chelating agent and the diluent, wherein the compound of formula (I) has a concentration of 0.002 mg/mL to 50 mg/mL, the surfactant has a concentration of 0.02 mg/mL to 3 mg/mL, the buffering agent has a concentration of 0.1 mg/mL to about 25 mg/mL, the osmotic pressure regulator has a concentration of 5 mg/mL to 9 mg/mL, and the metal chelating agent has a concentration of 0.01 mg/mL to about 5 mg/mL; the surfactant is one or more of Tween 20, Tween 80 and Span 20, the buffering agent is one or more of sodium dihydrogen phosphate or monohydrate thereof, disodium hydrogen phosphate, tartaric acid and citric acid, the osmotic pressure regulator is sodium chloride, the metal chelating agent is one or more of disodium edetate and calcium disodium edetate, and the diluent is water.

In some embodiments, the pharmaceutical composition comprises the compound of formula (I), Tween 80, sodium dihydrogen phosphate or monohydrate thereof, disodium hydrogen phosphate, sodium chloride, disodium edetate and water; wherein the compound of formula (I) has a concentration of 0.002 mg/mL to 50 mg/mL, Tween 80 has a concentration of 0.02 mg/mL to 3 mg/mL, sodium dihydrogen phosphate and disodium hydrogen phosphate have a concentration of 0.1 mg/mL to 25 mg/mL, sodium chloride has a concentration of 5 mg/mL to 9 mg/mL, and disodium edetate has a concentration of 0.01 mg/mL to about 5 mg/mL.

In some embodiments, in the pharmaceutical composition of the compound of formula (I) or the pharmaceutically acceptable salt thereof, the compound of formula (I) is in a solid form; in some embodiments, the compound of formula (I) is a crystalline form of the compound of formula (I).

In some embodiments, in the pharmaceutical composition of the compound of formula (I) or the pharmaceutically acceptable salt thereof, the compound of formula (I) is a product obtained by subjecting the crystalline form of the compound of formula (I) to particle size control.

In some embodiments, the crystalline form of the compound of formula (I) has diffraction peaks in an X-ray powder diffraction pattern using Cu Kα radiation at the following 2Θ: 5.81±0.2°, 13.96±0.2°, 15.01±0.2°, 17.95±0.2° and 24.73±0.2°.

In some embodiments of the present application, the crystalline form of the compound of formula (I) has diffraction peaks in an X-ray powder diffraction pattern using Cu Kα radiation at the following 2Θ: 5.81±0.2°, 8.38±0.2°, 11.16±0.2°, 13.96±0.2°, 14.47±0.2°, 15.01±0.2°, 17.95±0.2°, 24.73±0.2° and 26.13±0.2°.

In some embodiments, the crystalline form of the compound of formula (I) has diffraction peaks in an X-ray powder diffraction pattern using Cu Kα radiation at the following 2Θ: 5.81±0.2°, 8.38±0.2°, 11.16±0.2°, 13.96±0.2°, 14.47±0.2°, 15.01±0.2°, 16.76±0.2°, 17.95±0.2°, 20.83±0.2°, 24.73±0.2° and 26.13±0.2°.

In some embodiments, the crystalline form of the compound of formula (I) has diffraction peaks in an X-ray powder diffraction pattern using Cu Kα radiation at the following 2Θ: 5.81±0.2°, 8.38±0.2°, 9.13±0.2°, 11.16±0.2°, 11.60±0.2°, 12.82±0.2°, 13.96±0.2°, 14.47±0.2°, 15.01±0.2°, 16.76±0.2°, 17.95±0.2°, 18.91±0.2°, 20.83±0.2°, 24.36±0.2°, 24.73±0.2°, 25.78±0.2° and 26.13±0.2°.

In some embodiments, the crystalline form of the compound of formula (I) comprises 5, 6, 7, 8, 9, 10, 11, 12 or more diffraction peaks in an X-ray powder diffraction pattern using Cu Kα radiation selected from the following 2Θ angles: 5.81±0.2°, 8.38±0.2°, 9.13±0.2°, 11.16±0.2°, 11.60±0.2°, 12.82±0.2°, 13.96±0.2°, 14.47±0.2°, 15.01±0.2°, 16.76±0.2°, 17.95±0.2°, 18.91±0.2°, 20.83±0.2°, 24.36±0.2°, 24.73±0.2°, 25.78±0.2° and 26.13±0.2°. In some embodiments, the crystalline form of the compound of formula (I) comprises 5, 6, 7, 8, 9, 10 or 11 diffraction peaks in an X-ray powder diffraction pattern using Cu Kα radiation selected from the following 2Θ angles: 5.81±0.2°, 8.38±0.2°, 11.16±0.2°, 13.96±0.2°, 14.47±0.2°, 15.01±0.2°, 16.76±0.2°, 17.95±0.2°, 20.83±0.2°, 24.73±0.2° and 26.13±0.2°.

In some embodiments, the crystalline form of the compound of formula (I) comprises 5, 6, 7, 8 or 9 diffraction peaks in an X-ray powder diffraction pattern using Cu Kα radiation selected from the following 2Θ angles: 5.81±0.2°, 8.38±0.2°, 11.16±0.2°, 13.96±0.2°, 14.47±0.2°, 15.01±0.2°, 17.95±0.2°, 24.73±0.2° and 26.13±0.2°. In some embodiments, the crystalline form of the compound of formula (I) has diffraction peaks in an XRPD pattern using Cu Kα radiation with peak positions and relative intensities as shown in Table 1 below:

**Table 1**

| **No.** | **2θ** (±0.2°) | **Relative intensity (%)** | **No.** | **2θ** (±0.2°) | **Relative intensity (%)** |
|---|---|---|---|---|---|
| 1 | 5.81 | 47.9 | 17 | 17.69 | 20.9 |
| 2 | 8.38 | 23.7 | 18 | 17.95 | 69.7 |
| 3 | 9.13 | 15.4 | 19 | 18.91 | 15.0 |
| 4 | 10.57 | 8.2 | 20 | 20.21 | 8.0 |
| 5 | 11.16 | 46.9 | 21 | 20.83 | 24.3 |
| 6 | 11.60 | 17.6 | 22 | 21.25 | 5.3 |
| 7 | 11.77 | 6.2 | 23 | 22.96 | 9.9 |
| 8 | 12.82 | 16.5 | 24 | 24.14 | 5.1 |
| 9 | 13.96 | 99.5 | 25 | 24.36 | 19.5 |
| 10 | 14.47 | 38.4 | 26 | 24.73 | 100.0 |
| 11 | 15.01 | 80.7 | 27 | 25.48 | 11.1 |
| 12 | 15.71 | 8.0 | 28 | 25.78 | 19.9 |
| 13 | 16.03 | 9.1 | 29 | 26.13 | 61.8 |
| 14 | 16.54 | 9.2 | 30 | 29.05 | 12.5 |
| 15 | 16.76 | 24.4 | 31 | 29.37 | 6.3 |
| 16 | 17.47 | 8.3 | | | |

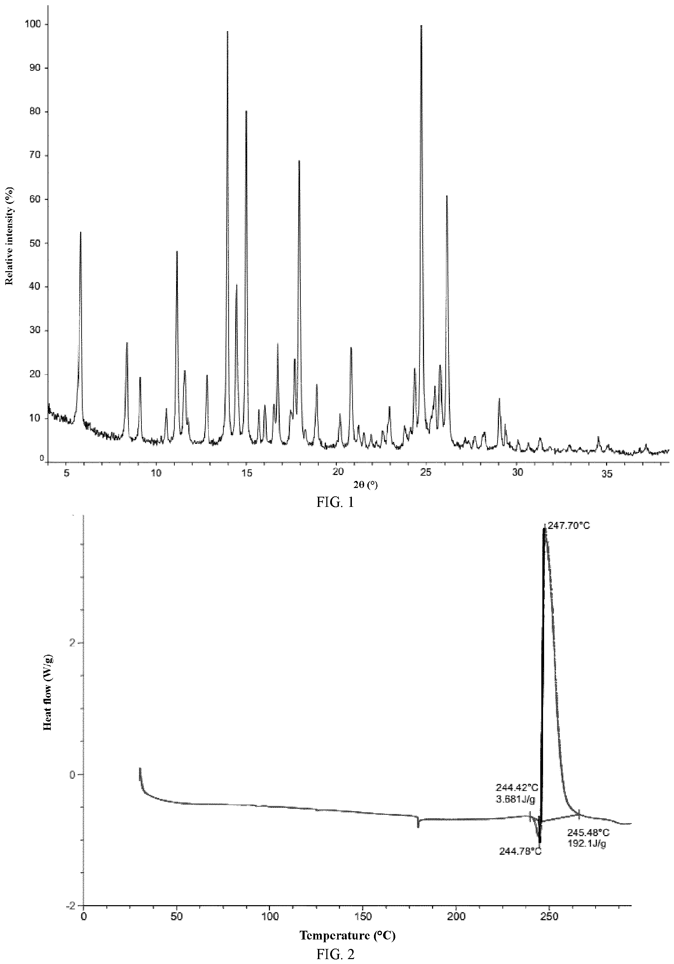

In some embodiments, the crystalline form of the compound of formula (I) has diffraction peaks in an XRPD pattern using Cu Kα radiation as shown in FIG. 1.

In some embodiments, the crystalline form of the compound of formula (I) has an exothermic peak in a differential scanning calorimetry curve at 247.70 °C ± 2 °C.

In some embodiments, the crystalline form of the compound of formula (I) has a differential scanning calorimetry pattern as shown in FIG. 2.

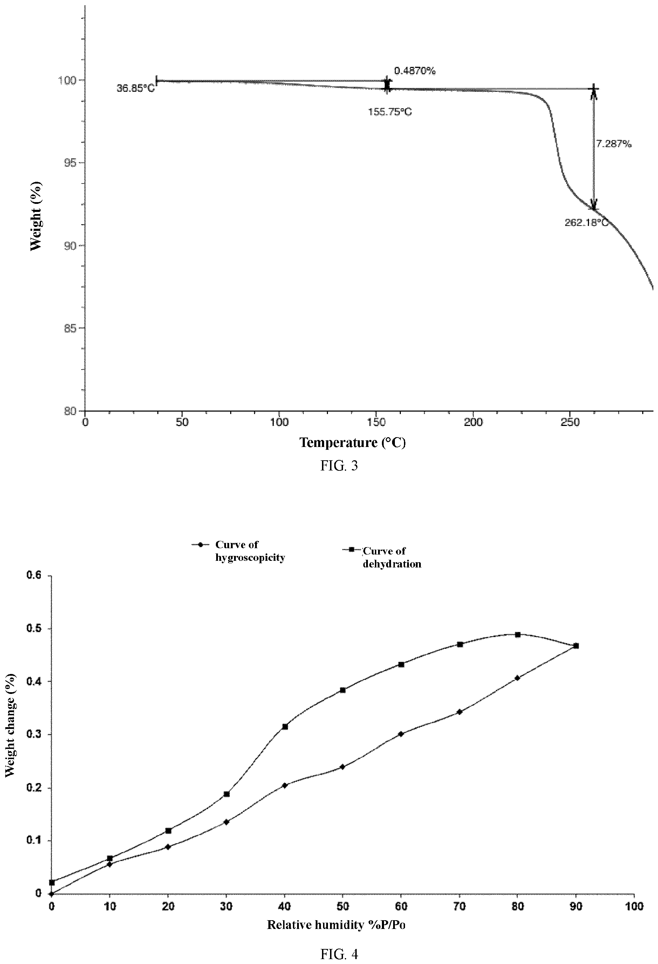

In some embodiments, the crystalline form of the compound of formula (I) has a weight loss of 0.4870% at 155.75±2 °C and a weight loss of 7.287% at 155.75±2 °C to 262.18±2 °C in a thermogravimetric analysis curve. In some embodiments, the crystalline form of the compound of formula (I) has a thermogravimetric analysis pattern as shown in FIG. 3.

In some embodiments, in the pharmaceutical composition, the compound of formula (I) or the pharmaceutically acceptable salt thereof has a particle size of X₅₀ < 10 µm, preferably between 0.1 µm and 8 µm.

In some embodiments, in the pharmaceutical composition, the compound of formula (I) or the pharmaceutically acceptable salt thereof has a particle size of X₅₀ ≤ 5 µm and X₉₀ ≤ 10 µm.

The pharmaceutical compound disclosed herein may be in a variety of dosage forms suitable for oral or inhalational administration to human, for example, a solution.

In some embodiments, the pharmaceutical composition disclosed herein is administered by inhalation.

In some embodiments, the pharmaceutical composition disclosed herein is administered by oral or nasal inhalation.

In some embodiments, the pharmaceutical composition disclosed herein is a solution for inhalation.

In some embodiments, the pharmaceutical composition disclosed herein is in the form of a suspension.

In some embodiments, the pharmaceutical composition disclosed herein is in the form of a suspension for inhalation.

In another aspect, the present application provides a method for preparing the pharmaceutical composition comprising: mixing the surfactant and the compound of formula (I) or the pharmaceutically acceptable salt thereof. Preferably, the method comprises mixing the surfactant, the compound of formula (I) or the pharmaceutically acceptable salt thereof, and at least one selected from the group consisting of: the metal chelating agent, the buffering agent, the diluent, and the osmotic pressure regulator. More preferably, the present application provides a method for preparing the pharmaceutical composition comprising: mixing the wetting agent, the buffering agent, the osmotic pressure regulator, the metal chelating agent, the compound of formula (I) or the pharmaceutically acceptable salt thereof, and the diluent.

In some embodiments, the method for preparing the pharmaceutical composition comprises:
1) mixing the wetting agent, the buffering agent, the osmotic pressure regulator, the metal chelating agent and the diluent to give a solution,
2) and mixing the compound of formula (I) or the pharmaceutically acceptable salt thereof with the solution in step 1).

In some embodiments, the method further comprises step 3): homogenizing the product obtained in step 2).

In some embodiments, after the homogenizing procedure, the compound of formula (I) or the pharmaceutically acceptable salt thereof has a particle size of X₅₀ ≤ 5 µm and X₉₀ < 10 µm.

In some embodiments, the method further comprises a filling step.

In yet another aspect, the present application further provides a method for preventing or treating a condition associated with PDE3 and/or PDE4 in a mammal, comprising administering to a mammal, preferably a human, in need thereof a therapeutically effective amount of the pharmaceutical composition.

In yet another aspect, the present application further provides use of the pharmaceutical composition in preparing a medicament for preventing or treating a condition associated with PDE3 and/or PDE4.

In yet another aspect, the present application further provides use of the pharmaceutical composition in preventing or treating a condition associated with PDE3 and/or PDE4.

In yet another aspect, the present application further provides the pharmaceutical composition for use in preventing or treating a condition associated with PDE3 and/or PDE4.

In some embodiments of the present application, the condition associated with PDE3 and/or PDE4 is selected from the group consisting of asthma and chronic obstructive pulmonary disease (COPD).

### Technical Effects

The compound of formula (I) and the pharmaceutical composition thereof disclosed herein have remarkable dual inhibitory effect on PDE3 and PDE4, have significant inhibitory effect on TNF-α in human peripheral blood mononuclear cells (hPBMCs), and also show excellent anti-inflammatory effect in rat acute lung injury model induced by lipopolysaccharide (LPS). The compound has high *in vivo* plasma clearance, low systemic exposure in plasma by oral administration and low oral bioavailability, and good safety in administration via a local route. Its inhibitory effect is low on 5 isozymes (CYP1A2, CYP2C9, CYP2C19, CYP2D6 and CYP3A4) of human liver microsomal cytochrome P450, and the risk of drug-drug interaction is avoided. Besides, the compound reduces the total white blood cells in BALF, has remarkable anti-inflammatory effect, takes effect at a low dose, and reduces the airway resistance index Penh.

The crystalline form of the compound of formula (I) and the pharmaceutically acceptable salt thereof of the present application have advantages in terms of pharmaceutical activity, pharmacokinetics, bioavailability, hygroscopicity, melting point, stability, solubility, purity, ease of preparation, etc., to meet the requirements of pharmaceutics in terms of production, storage, transportation, formulation, etc. The crystal form of the compound of formula (I) has low hygroscopicity, and is favorable for the absorption of inhaled compound.

The pharmaceutical composition of the compound of formula (I) has good stability without increase of impurities and shows pharmaceutically acceptable level of impurities. The pharmaceutical composition has good dispersibility and stable dynamics, demonstrated no significant particle precipitations and no increase in particle size, and is within the range required for effective delivery of approved inhalation products. In addition, the composition has uniform and moderate particle size and high absorption rate. The composition has good delivery rate, an accurate dosage, a high proportion of inhalable aerosol particles, and a high amount of inhalable fine particles.

### Definitions

Unless otherwise required in the present application, throughout the whole specification and the claims which follow, the word "comprise", and variants thereof such as "comprises" and "comprising", or equivalents shall be construed in an open-ended, inclusive sense, i.e., "includes but is not limit to", indicating that in addition to the listed elements, components and procedures, other unspecified elements, components and procedures may also be encompassed.

"One embodiment", "an embodiment", "in another embodiment" or "in some embodiments" used in the specification means that a specific reference element, structure or feature described in connection with the embodiment is included in at least one embodiment. Thus, the phrases "in one embodiment", "in an embodiment", "in another embodiment" and "in some embodiments" in various places throughout the specification are not necessarily all referring to the same embodiment. Furthermore, the specific elements, structures, or features may be combined in any suitable manner in one or more embodiments.

It should be understood that, unless otherwise specified clearly, the singular forms "a," "an," and "the" used in the specification and the appended claims of the present application include plural referents; in other words, singular terms encompass plural terms herein, and vice versa. Thus, for example, the mentioned reaction including "a catalyst" includes one catalyst, or two or more catalysts. It should be understood that, unless otherwise specified clearly, the term "or" is generally employed in its sense including "and/or".

The term "treat" or "treatment" means administering the compound or formulation described in the present application to ameliorate or eliminate a disease or one or more symptoms associated with the disease, and includes:
(i) inhibiting a disease or disease state, i.e., arresting its development; and
(ii) alleviating a disease or disease state, i.e., causing its regression.

The term "prevent" or "prevention" means administering the compound or formulation of the present application to prevent a disease or one or more symptoms associated with the disease, and includes: preventing the occurrence of the disease or disease state in a mammal, particularly when such a mammal is predisposed to the disease state but has not yet been diagnosed with it.

The term "therapeutically effective amount" refers to an amount of the compound of the present application for (i) treating or preventing a specific disease, condition or disorder; (ii) alleviating, ameliorating or eliminating one or more symptoms of a specific disease, condition or disorder, or (iii) preventing or delaying onset of one or more symptoms of a specific disease, condition or disorder described herein. The amount of the compound of the present application composing the "therapeutically effective amount" varies dependently on the compound, the disease state and its severity, the mode of administration, and the age of the mammal to be treated, but can be determined routinely by those skilled in the art in accordance with their knowledge and the present disclosure. Typically, the particle size is quantified by measuring the characteristic equivalent spherical diameter (referred to as the volume diameter) via laser diffraction, e.g., via a laser particle size analyzer.

In the present application, the particle size distribution is expressed in terms of volume diameter (VD).

The term "X₁₀" refers to the particle size corresponding to a cumulative volume distribution percentage of 10%, which physically means that particles with particle sizes less than it make up 10% of the total volume.

The term "X₅₀" refers to the particle size corresponding to a cumulative volume distribution percentage of 50%, which is referred to as the volume median diameter and physically means that particles with particle sizes less than it make up 50% of the total volume.

The term "X₉₀" refers to the particle size corresponding to a cumulative volume distribution percentage of 90%, which physically means that particles with particle sizes less than it make up 90% of the total volume.

Unless otherwise indicated herein, parameter values (including 2Θ values, reaction conditions) are to be construed as modified by the term "about" to reflect the measurement error and the like existing in the values, e.g., there is an error of ±5% relative to the given value.

All patents, patent applications and other identified publications are expressly incorporated herein by reference for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

### BRIEF DESCRIPTION OF THE DRAWINGS

FIG. 1 is an XRPD pattern of the crystalline form of the compound of formula (I);
FIG. 2 is a DSC pattern of the crystalline form of the compound of formula (I);
FIG. 3 is a TGA pattern of the crystalline form of the compound of formula (I);
FIG. 4 is a DVS pattern of the crystalline form of the compound of formula (I);
FIG. 5 shows the total number of white blood cells in BALF;
FIG. 6 shows the methacholine (Mch) challenge pulmonary function test (airway resistance index Penh).

### DETAILED DESCRIPTION

The following specific examples are intended to allow those skilled in the art to clearly understand and implement the present application. These specific examples should not be considered as limit to the scope of the present application, but merely as exemplary description and representative of the present application.

### Examples

### Synthesis of intermediate BB-1

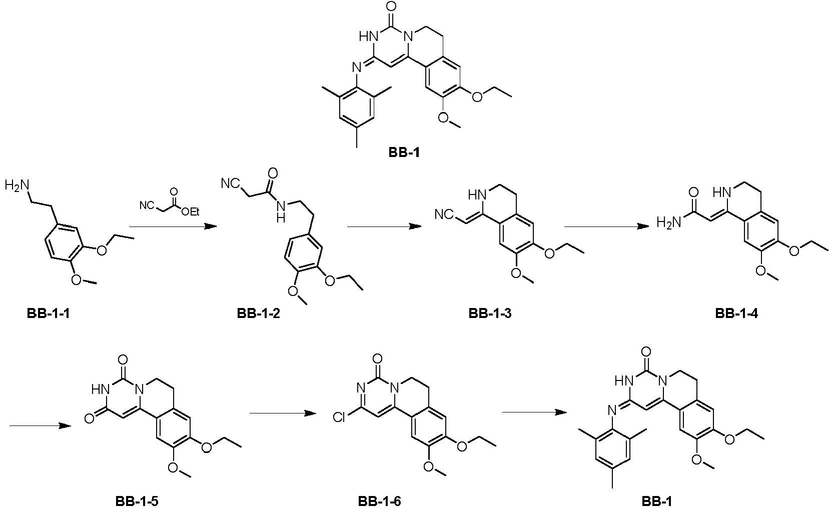

### Step 1: synthesis of compound BB-1-2

A mixture of compound **BB-1-1** (21.10 g) and ethyl cyanoacetate (11.00 g, 10.38 mL) was stirred at 100 °C for 16 h in nitrogen atmosphere. After completion of the reaction, the mixture was cooled to 70 °C, ethanol (30 mL) was slowly and dropwise added, and a large amount of solid was precipitated. The resulting mixture was filtered, and the filter cake was dried under reduced pressure to give product **BB-1-2.**

**¹H NMR (400MHz, DMSO-d6)** δ = 8.26 (t, *J* = 5.2 Hz, 1H), 6.86 (d, *J* = 8.0 Hz, 1H), 6.79 (br s, 1H), 6.71 (d, 8.0 Hz, 1H),4.00 (q, *J* = 6.8 Hz, 2H), 3.72 (s, 3H), 3.59 (s, 2H), 3.31 - 3.23 (m, 2H), 2.64 (t, *J* = 7.2 Hz, 2H), 1.32 (t, *J* = 6.8 Hz, 3H). **MS-ESI** *m*/*z:* 263.1[M+H]⁺.

### Step 2: synthesis of compound BB-1-3

Phosphorus oxychloride (379.50 g, 230.00 mL) was heated to 85 °C in nitrogen atmosphere and compound **BB-1-2** (26.00 g) was added in portions. The reaction mixture was stirred at 85 °C for 2 h. After the reaction was completed, most of the phosphorus oxychloride was removed by reduced pressure distillation. To the residue was added dichloromethane (200 mL) and the mixture was washed with water (100 mL × 2). The organic phase was dried over anhydrous sodium sulfate, filtered to remove the desiccant, and then concentrated under reduced pressure. The resulting crude product was purified by slurrying with ethyl acetate (20 mL) to give compound **BB-1-3.**

**¹H NMR (400MHz, CD₃OD)** δ = 7.16 (s, 1H), 6.83 (s, 1H), 4.62 (s, 1H), 4.12 (q, *J=* 6.8 Hz, 2H), 3.86 (s, 3H), 3.35 *(d, J=* 6.4 Hz, 2H), 2.84 *(t, J=* 6.4 Hz, 2H), 1.44 *(t, J=* 6.8 Hz, 3H). **MS-ESI *m*/*z***: 245.1[M+H]⁺.

### Step 3: synthesis of compound BB-1-4

Compound **BB-1-3** (1.00 g) was added to 98% concentrated sulfuric acid (12.88 g, 128.69 mmol, 7.00 mL) in portions at 0 °C. The reaction mixture was stirred at 27 °C for 3 h. After the reaction was completed, the mixture was added to cold water (15 mL), and then aqueous sodium hydroxide solution (4 mol/L, 32 mL) was added dropwise to adjust the pH to neutral, followed by extraction with ethyl acetate (100 mL × 3). Then the organic phases were combined, dried over anhydrous sodium sulfate, filtered to remove the desiccant, and concentrated under reduced pressure to give compound **BB-1-4.**

**MS-ESI m/z:** 263.1[M+H]⁺.

### Step 4: synthesis of compound BB-1-5

Sodium (2.42 g) was added in portions to ethanol (80 mL) at 0 °C. After the mixture was stirred at 28 °C for 0.5 h, compound **BB-1-4** (6.90 g) was added to the solution in portions, and the mixture was stirred at 80 °C for 0.5 h. Then, diethyl carbonate (9.32 g, 9.51 mL) was added in one portion, and the mixture was stirred for 5 h at 80 °C. After the reaction was completed, the mixture was cooled to room temperature, ice water (30 mL) was slowly added, and then diluted hydrochloric acid (2 mol/L, 53 mL) was added to adjust the mixture to a neutral pH. A large amount of solid was precipitated. The mixture was filtered, and the resulting filter cake was purified by slurrying with ethanol (10 mL) to give compound **BB-1-5.**

**¹H NMR (400MHz, DMSO-d₆)** δ = 11.22 (br s, 1H), 7.35 (s, 1H), 6.95 (s, 1H), 6.22 (s, 1H), 4.09 (q, *J =* 6.8 Hz, 2H), 3.90 (br s, 2H), 3.83 (s, 3H), 2.89 (br s, 2H), 1.35 (t, *J =* 6.8 Hz, 3H). **MS-ESI** *m*/*z:* 289.1[M+H]⁺.

### Step 5: synthesis of compound BB-1-6

Compound **BB-1-5** (5.00 g) was dissolved in phosphorus oxychloride (30 mL) at room temperature. The reaction mixture was stirred at 100 °C for 16 h in nitrogen atmosphere. After the reaction was completed, most of the solvent was removed by reduced pressure distillation. Water (100 mL) was added and the resulting mixture was extracted with dichloromethane (150 mL × 2). The organic phases were combined, dried over anhydrous sodium sulfate, filtered to remove the desiccant, and concentrated under reduced pressure to give compound **BB-1-6. MS-ESI *m*/*z:*** 306.9[M+H]⁺.

### Step 6: synthesis of compound BB-1

Compound **BB-1-6** (925.67 mg) was dissolved in isopropanol (8 mL) at room temperature, and then 2,4,6-trimethylaniline (2.10 g) was added. The reaction mixture was stirred at 90 °C for 15 h in nitrogen atmosphere. After the reaction was completed, the mixture was cooled to room temperature and concentrated under reduced pressure, and the resulting residue was purified by slurrying with ethanol (6 mL) to give compound **BB-1.**

**¹H NMR (400MHz, DMSO-d₆)** δ = 8.85 (br s, 1H), 7.27 (s, 1H), 6.97 (s, 1H), 6.90 (s, 2H), 6.45 (s, 1H), 4.10 (q, *J* = 6.8 Hz, 2H), 3.90 (t, *J* = 6.0 Hz, 2H), 3.86 (s, 3H), 2.87 (t, *J* = 6.0 Hz, 2H), 2.45 (s, 3H), 2.11 (s, 6H), 1.37 (t, *J* = 6.8 Hz, 3H). MS-ESI *m*/*z:* 406.2[M+H]⁺.

### Synthesis of compound BB-4

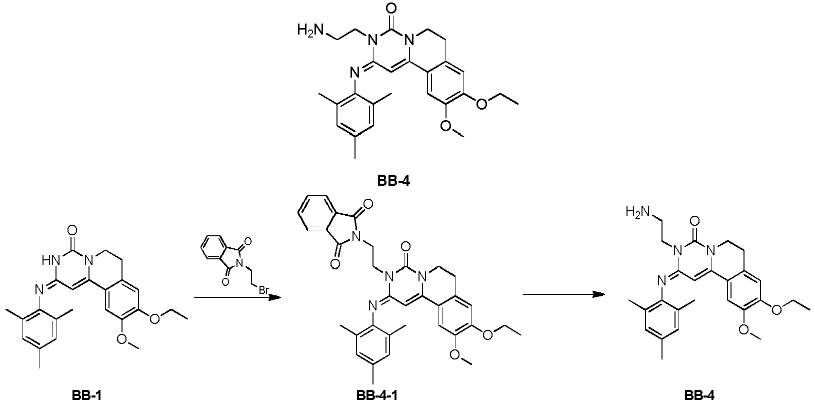

### Step 1: synthesis of compound BB-4-1

Compound **BB-1** (1.00 g) was dissolved in 2-butanone (35 mL) at room temperature, and 2-(2-bromoethyl)isoindoline-1,3-dione (3.76 g), potassium carbonate (3.07 g) and sodium iodide (2.22 g) were added successively. The reaction mixture was stirred at 85 °C for 72 h in nitrogen atmosphere. After the reaction was completed, the mixture was concentrated to remove most of the organic solvent before water (30 mL) and ethyl acetate (25 mL × 3) were added for extraction. The organic phases were combined, dried over anhydrous sodium sulfate, filtered to remove the desiccant and concentrated under reduced pressure. The resulting residue was purified by flash silica gel column chromatography (eluent: petroleum ether/ethyl acetate = 15/1-3/1) to give compound **BB-4-1.**

**MS-ESI *m*/*z:*** 579.3[M+H]⁺.

### Step 2: synthesis of compound BB-4

Compound **BB-4-1** (500.00 mg) was dissolved in trichloromethane (3 mL) and ethanol (3 mL) at room temperature, and hydrazine hydrate (152.67 mg, 85% purity) was added. The mixture was stirred at 28 °C for 16 h in nitrogen atmosphere. After the reaction was completed, the mixture was concentrated to remove most of the organic solvent before water (15 mL) and dichloromethane (15 mL × 3) were added for extraction. Then the organic phases were combined, dried over anhydrous sodium sulfate, filtered to remove the desiccant, and concentrated under reduced pressure to give compound **BB-4.**

**¹H NMR (400MHz, DMSO-d₆)** δ= 6.95 (s, 1H), 6.85 (br s, 2H), 6.66 (s, 1H), 5.31 (s, 1H), 4.14 (t, *J* = 6.8 Hz, 2H), 4.05 (q, *J =* 6.8 Hz, 2H), 3.91 (t, *J =* 6.4 Hz, 2H), 3.62 (s, 3H), 2.90 - 2.86 (m, 4H), 2.22 (s, 3H), 1.95 (br s, 6H), 1.33 (t, *J* = 6.8 Hz, 3H). **MS-ESI *m*/*z***: 449.2[M+H]⁺.

### Example 1: Preparation of compound of formula (I)

5-Hydroxy-3-methyl-1,2,3-triazole-4-carboxylic acid (18.50 mg) was dissolved in DCM (1 mL) at 20 °C. HATU (8.80 mg) and triethylamine (57.40 µL) were added and the mixture was stirred for 2 h, followed by addition of compound **BB-4** (50 mg) and stirring at the temperature for 16 h. The mixture was diluted to 10 mL with DCM, washed with water (30 mL × 3), dried over anhydrous sodium sulfate, and filtered to remove the desiccant, and the filtrate was concentrated under reduced pressure to remove the solvent to give a crude product. The crude product was separated and purified by prep-HPLC to give the target compound of formula (I) in the form of a yellow solid.

**¹H NMR (400 MHz, CD₃OD)** δ = 6.94 (s, 2H), 6.87 (s, 1H), 6.77 (s, 1H), 5.52 (s, 1H), 4.48 (t, *J =* 6.0 Hz, 2H), 4.15 (s, 3H), 4.12 - 4.08 (m, 2H), 4.01 (t, *J* = 6.0 Hz, 2H), 3.87 (t, *J =* 6.0 Hz, 2H), 3.69 (s, 3H), 2.94 (t, *J =* 6.0 Hz, 2H), 2.29 (s, 3H), 2.06 (s, 6H), 1.41 (t, *J* = 6.8 Hz, 3H). MS m/z [M+H]⁺ 574.1.

### Example 2: Preparation of crystalline form of compound of formula (I)

50 mg of the compound of formula (I) was added to a 4-mL glass bottle, 1 mL of anhydrous ethanol and 0.2 mL of water were added, and the mixture was heated to 40 °C and stirred for 48 h. The mixture was naturally cooled to room temperature, centrifuged to separate the solid, and dried in vacuum to give 46 mg of solid crystalline form. The XRPD pattern is shown in FIG. 1, the DSC pattern is shown in FIG. 2, and the TGA pattern is shown in FIG. 3.

### Examples 3-36

Procedures:
1) various excipients were added to a preparation tank, and the mixture was stirred for dissolving to give an excipient solution;
2) the compound of formula (I) was added to the prepared excipient solution, and the mixture was stirred to form a uniform suspension;
3) the suspension was further subjected to a high-pressure homogenizer, a microjet, or a sand mill, and the like , and the particle size of the compound of formula (I) in the preparation was controlled at X₅₀ ≤ 5 µm and X₉₀ < 10 µm; and
4) a product was obtained.

The amounts of the specific excipients and the products are shown in the following Table 2.

**Table 2**

| Example No. | Composition of excipient solution | | | | | | | | | | | Concentration of compound of formula (I) in the product (mg/mL) |
|---|---|---|---|---|---|---|---|---|---|---|---|---|
| | Tween 20 (g) | Tween 80 (g) | Span 20 (g) | Disodium hydrogen phosphate (g) | Sodium dihydrogen phosphate (g) | Tartaric acid (g) | Citric acid (g) | Sodium chloride (g) | Disodium edetate (g) | Calcium disodium edetate (g) | Water (mL) | |
| 3 | 0.5 | / | / | / | / | / | / | / | / | / | 1000 | 1 |
| 4 | / | 0.5 | / | / | / | / | / | / | / | / | 1000 | 1 |
| 5 | / | / | 0.5 | / | / | / | / | / | / | / | 1000 | 1 |
| 6 | 0.5 | / | 0.05 | / | / | / | / | / | / | / | 1000 | 1 |
| 7 | / | 0.5 | 0.05 | / | / | / | / | / | / | / | 1000 | 1 |
| 8 | 0.5 | | 0.05 | 2 | 4 | / | / | / | / | / | 1000 | 1 |
| 9 | / | 0.5 | / | 2 | 4 | / | / | / | / | / | 1000 | 1 |
| 10 | / | 0.5 | / | 2 | 4 | / | / | / | 2 | / | 1000 | 1 |
| 11 | 0.5 | / | / | 2 | 4 | / | / | / | 2 | / | 1000 | 1 |
| 12 | 0.5 | / | 0.05 | 2 | 4 | / | / | / | 2 | / | 1000 | 1 |
| 13 | / | 0.5 | 0.05 | 2 | 4 | / | / | / | 2 | / | 1000 | 1 |
| 14 | 0.5 | 0.5 | 0.05 | 2 | 4 | / | / | / | 2 | / | 1000 | 1 |
| 15 | 0.5 | 0.5 | 0.05 | 2 | 4 | / | / | 6.5 | 2 | / | 1000 | 1 |
| 16 | / | 0.5 | / | 2 | 4 | / | / | 6.5 | 2 | / | 1000 | 1 |
| 17 | 0.05 | / | 0.01 | 2 | 4 | / | / | 6.5 | / | / | 1000 | 0.02 |
| 18 | 0.5 | / | 0.05 | 2 | 4 | / | / | 6.5 | / | / | 1000 | 6 |
| 19 | 2 | / | 1 | 12.8 | / | / | / | / | / | / | 1000 | 50 |
| 20 | 2 | / | 1 | 2 | 4 | / | / | / | / | / | 1000 | 50 |
| 21 | / | 0.05 | 0.01 | 2 | 4 | / | / | 6.5 | / | / | 1000 | 0.02 |
| 22 | / | 0.5 | 0.05 | 2 | 4 | / | / | 6.5 | / | / | 1000 | 6 |
| 23 | / | 2 | 1 | 12.8 | / | / | / | / | / | / | 1000 | 50 |
| 24 | / | 2 | 1 | 1.4 | 3.6 | / | / | 8.5 | / | / | 1000 | 50 |
| 25 | / | 0.1 | / | 2 | 4 | / | / | 6.5 | 0.01 | / | 1000 | 0.002 |
| 26 | / | 0.3 | / | 2 | 4 | / | / | 6.5 | 0.5 | / | 1000 | 0.002 |
| 27 | / | 1 | / | 1.4 | 3.6 | / | / | 8.5 | 1.5 | / | 1000 | 1 |
| 28 | / | 0.1 | / | 2 | 4 | / | / | 8.5 | 0.1 | / | 1000 | 1 |
| 29 | / | 2 | / | 0.7 | 1.7 | / | / | 9 | 2 | / | 1000 | 50 |
| 30 | / | 0.02 | / | 2 | 4 | / | / | 6.5 | 0.01 | / | 1000 | 1 |
| 31 | / | 0.02 | / | 2 | 4 | / | / | 6.5 | 0.03 | / | 1000 | 1 |
| 32 | 0.2 | 0.2 | / | 2 | 4 | / | / | 6.5 | 0.02 | / | 1000 | 1 |
| 33 | / | 2 | / | 0.7 | 1.7 | / | / | 9 | / | 2 | 1000 | 50 |
| 34 | / | 2 | / | 0.7 | 1.7 | / | / | / | 1 | 1 | 1000 | 50 |
| 35* | / | 2 | / | / | / | 3 | | 8 | 1 | / | 1000 | 50 |
| 36* | / | 2 | / | / | / | / | 3 | 8 | 1 | / | 1000 | 50 |

| | | | | | | | | | | | | |
|---|---|---|---|---|---|---|---|---|---|---|---|---|
| * In Example 35 and Example 36, after mixing and dissolving the various excipients in the step 1), an appropriate amount of sodium hydroxide was added to adjust to pH 5.0 to 7.0. | | | | | | | | | | | | |

### Experimental Example 1: Stability study of solid crystalline form of the compound of formula (I) High performance liquid chromatography (HPLC)

The chromatographic conditions of the HPLC method are seen in table below:
Chromatography column: Zorbax SB C-18, 4.6 mm × 150 mm, 5 µm (PDS-HPLC-007)
Mobile phase A: 0.1% TFA in water
Mobile phase B: 100% ACN
Preparation of sample: the sample was dissolved with a mixed solvent of acetonitrile and water (acetonitrile:water = 50:50 (v/v))

### Solid stability stakeout method

The stability of the compound in the following conditions was examined, and samples were taken at different time points to detect the content. About 5 mg of the crystalline form of the compound of formula (I) prepared in Example 2 was accurately weighted in duplicate, transferred to a dry and clean glass bottle, spread into a thin layer as test samples, and placed in experimental conditions of influential factors ((60 °C), (relative humidity 92.5%), illumination (total illumination of 1.2 × 10⁶ Lux•hr/near UV energy of 200 w•hr/m²), (40 °C, relative humidity 75%), or (60 °C, relative humidity 75%)). The samples were covered with aluminum foils having holes, and thus completely exposed to the conditions. Sampling analysis was performed at 5 days, 10 days, 1 month, 2 months and 3 months. The samples were completely exposed to illumination (visible light of 1200000 Lux, UV of 200 W) at room temperature. The experimental results are shown in Table 3.

**Table 3. Results of solid stability sample content assay (5 days, 10 days, 1 month, 2 months, 3 months**

| **RRT/Norm%** | **Total impurities %** |
|---|---|
| Day 0 | 0.11 |
| 60 °C - 5 days | 0.10 |
| 60 °C - 10 days | 0.11 |
| 92.5% RH - 5 days | 0.11 |
| 92.5% RH - 10 days | 0.10 |
| In the dark | 0.11 |
| Illumination | 0.10 |
| 40 °C + 75% RH - 10 days | 0.10 |
| 60 °C + 75% RH - 10 days | 0.10 |
| 40 °C + 75% RH - 1 month | 0.10 |
| 60 °C + 75% RH - 1 month | 0.10 |
| 40 °C + 75% RH - 2 months | 0.08 |
| 40 °C + 75% RH - 3 months | 0.09 |

As can be seen, the crystalline form of the compound of formula (I) of the present application has good stability in the conditions of high temperature, high humidity or illumination without the increase of impurities during the test.

### Experimental Example 2: Hygroscopicity study of crystalline form of the compound of formula (I)

Instrument model: SMS DVS Advantage
Test conditions: the sample (10-20 mg, the crystalline form prepared in Example 3) was placed in DVS sample tray for testing.
The detailed DVS parameters are as follows:
   Temperature: 25 °C
   Balancing: dm/dt = 0.01%/min (shortest: 10 min, longest: 180 min)
   Drying: drying at 0% RH for 120 min
   RH (%) test gradient: 10%
   Range of RH (%) test gradient: 0%-90%-0%. The resulting dynamic vapor sorption (DVS) plot is shown in FIG. 4.

As can be seen from FIG. 4, the crystalline form of the compound of formula (I) of the present application has a small hygroscopicity.

### Experimental Example 3: In vitro detection of the inhibitory activity of the compound against PDE 3A enzyme

**Objective:** to determine the AMP/GMP expression based on fluorescence polarization, i.e., to trace binding of AMP/GMP to antibody so as to indicate enzyme activity.

### Reagents:

**Buffer solution:** 10 mM Tris-HCl (pH 7.5), 5 mM MgCl₂, 0.01% Brij 35, 1 mM dithiothreitol (DTT), and 1% DMSO.

**Enzyme:** recombinant human PDE3A (Gene accession number: NM_000921; amino acid 669-end) was expressed by baculovirus in Sf9 insect cells using an N-terminal GST tag, with the molecular weight being 84 kDa.

**Enzyme substrate:** 1 µM cAMP

**Detection:** Transcreener^{®}AMP2/GMP2 antibody and AMP2/GMP2 AlexaFluor633 tracer.

### Procedures:

1. The recombinant human PDE3A enzyme and enzyme substrate (1 µM cAMP) were each dissolved in newly prepared experimental buffer solution;
2. The PDE3A enzyme buffer solution was transferred into reaction wells;
3. The compound which was dissolved in 100% DMSO was added to the reaction wells containing PDE3A enzyme buffer solution by acoustic technique (echo 550; millilambda range) and the mixture was incubated for 10 min at room temperature;
4. The enzyme substrate buffer solution was added to the above reaction wells to initiate the reaction;
5. The resulting mixture was incubated at room temperature for 1 h;
6. The detection mixture (Transcreener^{®}AMP2/GMP2 antibody and AMP2/GMP2 AlexaFluor633 tracer) was added to stop the reaction, and the resulting mixture was incubated for 90 min while slowly mixing. The measurement range of fluorescence polarization was Ex/Em = 620/688.

**Data analysis:** the fluorescence polarization signal was converted to nM based on AMP/GMP standard curve and the percentage enzyme activity relative to DMSO control calculated by Excel. GraphPad Prism was used for curve fitting (drawing medical icon). The results are shown in Table 4.

### Experimental Example 4: In vitro detection of the inhibitory activity of the compound against PDE 4B enzyme

**Objective:** to determine the AMP/GMP expression based on fluorescence polarization, i.e., to trace binding of AMP/GMP to antibody so as to indicate enzyme activity.

### Reagents:

**Buffer solution:** 10 mM Tris-HCl (pH 7.5), 5 mM MgCl₂, 0.01% Brij 35, 1 mM DTT, and 1% DMSO.

**Enzyme:** recombinant human PDE4B (Gene accession number: NM_002600; amino acid 305-end) was expressed by baculovirus in Sf9 insect cells using an N-terminal GST tag, with molecular weight being 78 kDa.

**Enzyme substrate:** 1 µM cAMP

**Detection:** Transcreener^{®}AMP2/GMP2 antibody and AMP2/GMP2 AlexaFluor633 tracer.

### Procedures:

1. The recombinant human PDE4B enzyme and enzyme substrate (1 µM cAMP) were each dissolved in newly prepared buffer solution.
2. The PDE4B enzyme buffer solution was transferred into reaction wells.
3. The compound dissolved in 100% DMSO was added to the reaction wells containing PDE4B enzyme buffer solution by acoustic technique (echo 550; millilambda range) and the mixture was incubated for 10 min at room temperature.
4. The enzyme substrate buffer solution was added to the above reaction wells to initiate reaction.
5. The resulting mixture was incubated at room temperature for 1 h.
6. The detection mixture (Transcreener^{®}AMP2/GMP2 antibody and AMP2/GMP2 AlexaFluor633 tracer) was added to stop the reaction, and the resulting mixture was incubated for 90 min while slowly mixing. The measurement range of fluorescence polarization was Ex/Em = 620/688.

**Data analysis:** the fluorescence polarization signal was converted to nM based on AMP/GMP standard curve and the percentage enzyme activity relative to DMSO control calculated by Excel. GraphPad Prism was used for curve fitting (drawing medical icon).

The results are shown in Table 4:

**Table 4. Results of in vitro screening test for the compound**

| **Compound** | **PDE3A** IC₅₀(nM) | **PDE4B** IC₅₀(nM) |
|---|---|---|
| Compound of formula (I) | 0.03 | 0.41 |

The active ingredient in the pharmaceutical composition of the present application has significant dual inhibitory effect on PDE3 and PDE4.

### Experimental Example 5: Pharmacokinetic study in beagle dogs

In this study, male beagle dogs were selected as test animals, and LC-MS/MS was used for quantitatively measuring the drug concentration in plasma of beagle dogs at different time points after intravenous injection or intragastric administration of the compound of formula (I) so as to evaluate the pharmacokinetics of the compound of formula (I) in beagle dogs.

The clear solution of the compound of formula (I) was injected into two beagle dogs of 10-12 kg via the cephalic vein or saphenous vein, and the clear solution of the compound of formula (I) was administered intragastrically to two beagle dogs of 10-12 kg (overnight fasted). The animals were all subjected to approximately 500-µL blood collection each time from peripheral veins at 0.0333, 0.0833, 0.25, 0.5, 1, 2, 4, 6, 8 and 24 h post-dose, and the blood was transferred into commercial centrifuge tubes containing 0.85-1.15 mg of K₂ EDTA·2H₂O anticoagulant, and plasma was separated by centrifugation at 3000 g for 10 min at 4 °C. The plasma concentration was measured by LC-MS/MS, and the relevant pharmacokinetic parameters were calculated using WinNonlin^{™} Version 6.3 (Pharsight, Mountain View, CA), a pharmacokinetic software, with a non-compartmental model linear-log trapezoidal method.

**Table 5. Pharmacokinetic parameters of the compound in beagle dogs**

| Pharmacokinetics in beagle dogs | Intravenous injection (0.5 mg/kg) | | | Intragastric administration (3 mg/kg) | | | |
|---|---|---|---|---|---|---|---|
| | Plasma clearance (mL/min/kg) | Half-life (h) | Area under plasma concentration-time curve (0-inf, nM.h) | Peak concentration (nM) | Time to peak (h) | Area under plasma concentration-time curve (0-inf, nM.h) | Bioavailability (%) |
| Compound of formula (1) | 70.3 | 0.3 | 210 | 59.4 | 0.6 | 123 | 7.5 |

The active ingredient in the pharmaceutical composition of the present application has high *in vivo* plasma clearance, low systemic exposure in plasma by oral administration and low oral bioavailability.

### Experimental Example 6: Inhibitory effect on activity of isoenzymes (CYP1A2, CYP2C9, CYP2C19, CYP2D6 and CYP3A4) of human liver microsomal cytochrome P450

A total of 5 specific probe substrates of 5 isoenzymes of CYP, namely phenacetin (CYP1A2), diclofenac (CYP2C9), (S)-mephenytoin (CYP2C19), dextromethorphan (CYP2D6) and midazolam (CYP3A4) were each co-incubated with human liver microsomes and the compound of formula (I), and then reduced nicotinamide adenine dinucleotide phosphate (NADPH) was added to initiate the reaction. After the reaction was completed, the samples were treated, and the concentrations of 5 metabolites (acetaminophen, 4'-hydroxydiclofenac, 4'-hydroxymephenytoin, dextrorphan and 1'-hydroxymidazolam) generated from the specific substrates were quantitatively detected by LC-MS/MS to calculate the corresponding half maximal inhibitory concentrations (IC 50).

**Table 6. Inhibitory effect of compound of formula (I) on five CYP enzymes**

| Compound No. | IC₅₀ (µM) | | | | |
|---|---|---|---|---|---|
| | CYP1A2 | CYP2C9 | CYP2C19 | CYP2D6 | CYP3A4 |
| Compound of formula (I) | 50 | 31 | 50 | 50 | 50 |

The active ingredient in the pharmaceutical composition of the present application has low inhibitory effect on the 5 isoenzymes (CYP1A2, CYP2C9, CYP2C19, CYP2D6 and CYP3A4) of human liver microsomal cytochrome P450.

### Experimental Example 7: Pharmacodynamic study in cigarette smoke-induced rat acute lung injury model Animals

Male Sprague-Dawley rats (supplied by Shanghai SLAC Laboratory Animal Co., Ltd.), SPF grade, approximately 200 g.

### Procedures

1. Animals were randomly divided into 6 groups according to body weight after arrival and a one-week acclimation;
2. On days 1-3 of the experiment, the corresponding compound of each group was atomized for 30 min. Then the animals in the model group and the treatment groups were exposed to cigarette smoke for 1 h, and after a 4-h interval, the animals were exposed to cigarette smoke again for 1 h. Cigarette smoke was given twice daily for 3 consecutive days. The control group animals were exposed to room air;
3. On day 4 of the experiment, the corresponding compound of each group was atomized for 30 min, and then the animals in the model group and the treatment groups were given the atomized 150 µg/mL LPS for 15 min by inhalation. After 3 h (from the time starting atomization), the animals were exposed to cigarette smoke for 1 h, and then the lung function (Penh and F) of the animals was examined; bronchoalveolar lavage fluid (BALF) was collected for cell counting after the animals were euthanized with CO₂.
4. Administration

Administration mode: the test compound and reference compound were given by atomization at the maximum atomization rate (approximately 12 mL) with the whole-body exposure atomization device for 30 min. Administration frequency: the drug or solvent were given by atomization for 30 min in every morning before exposure to cigarette smoke, and were given before the inhalation of the atomized LPS on day 4.

### 5. Measurements of pharmacodynamic endpoints

(1) Total white blood cells in BALF (bronchoalveolar lavage fluid);
(2) Mch challenge pulmonary function test (airway resistance index Penh);

### Table 7. Grouping

| Group | Number of animals | Compound concentration in solution for atomization | Time of administration |
|---|---|---|---|
| Model group | 10 | - | 30 min before the first cigarette smoke exposure every day |
| Low dose group of the compound of formula (I) | 10 | 0.05 mg/mL | 30 min before the first cigarette smoke exposure every day |
| High dose group of the compound of formula (I) | 10 | 0.15 mg/mL | 30 min before the first cigarette smoke exposure every day |

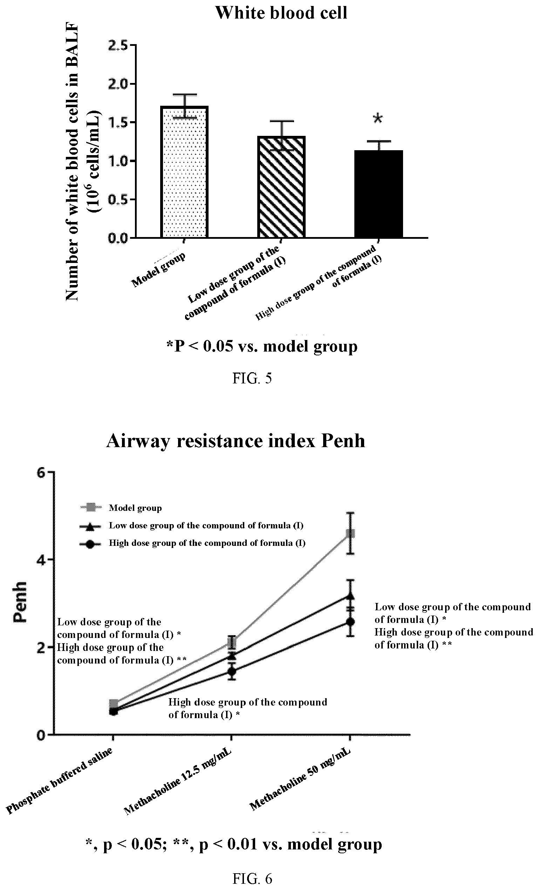

The experimental results are shown in FIG. 5 and FIG. 6.

As can be seen from FIG. 5 and FIG. 6, the active ingredient in the pharmaceutical composition of the present application can effectively reduce the number of white blood cells in the bronchoalveolar lavage fluid and the airway resistance index Penh.

### Experimental Example 8: In vitro detection of the inhibitory activity of the compound against TNF-α in human peripheral blood mononuclear cells

**Objective:** to measure the anti-inflammatory activity at cellular level of the test compound based on the level of TNF-α in human peripheral blood mononuclear cells (hPBMCs).

### Procedures:

1. Normal human whole blood was collected into an EDTA anticoagulation tube;
2. The PBMC was separated by Ficoll density gradient centrifugation and counted, and the cell concentration was adjusted to 2 × 10⁶ cells/mL;
3. To each well of a U-bottom 96-well plate were added 2 × 10⁵ cells, 1 ng/mL LPS, and solutions of the compound of formula (I) in DMSO at concentrations of 100 µM, 10 µM, 1 µM, 100 nM, 10 nM, 1 nM, 100 pM and 10 pM, with a system volume of 200 µL per well;
4. The mixture was incubated for 24 h, and then the supernatant was collected;
5. The level of TNF-α in the supernatant was detected by ELISA, an inhibition curve was fitted using Graphpad Prism software, and the IC₅₀ was calculated.

The results are shown in Table 8:

**Table 8. Results of in vitro test for the compound**

| **Compound** | **hPBMC** IC₅₀(nM) |
|---|---|
| Compound of formula (I) | 29.18 |

Therefore, the active ingredient in the pharmaceutical composition of the present application shows potent anti-inflammatory activity, and has significant inhibitory effect on TNF-α in human peripheral blood mononuclear cells (hPBMCs).

### Experimental Example 9: Stability Test

The product obtained in Example 28 was placed for 6 months in an accelerating condition (40 °C ± 2 °C/RH 25% ± 5%) and a long-term condition (30 °C ± 2 °C/RH 65% ± 5%), and the results are shown in Table 10.

**Table 9**

| Items | | Example 28 | | |
|---|---|---|---|---|
| | | Month 0 | Accelerated for 6 months | Long-term for 6 months |
| Related substance (%) | Total impurities | 0.71 | 0.94 | 0.98 |
| Particle size (µm) | X₁₀ | 0.7 | 0.7 | 0.7 |
| | X₅₀ | 1.4 | 1.5 | 1.5 |
| | X₉₀ | 2.5 | 2.9 | 2.6 |

As can be seen, the pharmaceutical composition of the present application shows good stability in the accelerating condition and the long-term condition without significant increase of impurities and particle size.

## Claims

1. A pharmaceutical composition, comprising: a compound of formula (I) or a pharmaceutically acceptable salt thereof, and a surfactant,

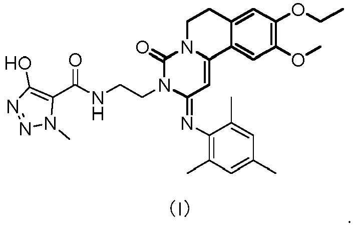

2. The pharmaceutical composition according to claim 1, comprising the compound of formula (I) or the pharmaceutically acceptable salt thereof, the surfactant, and at least one of a buffering agent, an osmotic pressure regulator, a metal chelating agent and a diluent.

3. The pharmaceutical composition claim 1 or 2, wherein the surfactant is a non-ionic surfactant;
or, the surfactant is one or more selected from the group consisting of polyoxyethylene glycol, polypropylene glycol alkyl ether, alkyl polyglucoside, octylphenol polyoxyethylene ether, alkylphenol polyoxyethylene ether, glycerin alkyl ester, polyoxyethylene sorbitan fatty acid ester, sorbitan alkyl ester, sorbitan fatty acid ester, cocamide MEA, cocamide DEA, dodecyldimethylamine oxide, a block copolymer of polyethylene glycol and polypropylene glycol, and polyethoxylated tallow amine;
or, the surfactant is one or more selected from the group consisting of a Tween and a Span;
or, the surfactant is one or more selected from the group consisting of Tween 20, Tween 80 and Span 20; or
or, the surfactant has a concentration of about 0.01 mg/mL to about 8 mg/mL.

4. The pharmaceutical composition according to claim 2, wherein the buffering agent is one or more selected from the group consisting of sulfuric acid, hydrochloric acid, sodium hydroxide, citric acid, sodium citrate, lactic acid, sodium lactate, acetic acid, sodium acetate, trisodium phosphate, sodium dihydrogen phosphate, disodium hydrogen phosphate, potassium dihydrogen phosphate, tartaric acid, sodium tartrate, glycine, boric acid and phthalic acid;
or, the buffering agent is selected from the group consisting of citric acid, a citrate, tartaric acid, a tartrate, phosphoric acid and a phosphate;
or, the buffering agent is selected from the group consisting of a citrate, a tartrate and a phosphate;
or, the buffering agent has a concentration of about 0.01 mg/mL to about 50 mg/mL;
or, the buffering agent is used to control a pH of the pharmaceutical composition between about 3.0 and about 8.5.

5. The pharmaceutical composition according to claim 2, wherein the osmotic pressure regulator is one or more selected from the group consisting of sodium chloride, potassium chloride, glucose, mannitol and xylitol.

6. The pharmaceutical composition according to claim 2, wherein the metal chelating agent is one or more selected from the group consisting of edetic acid, disodium edetate and calcium disodium edetate.

7. The pharmaceutical composition according to claim 2, wherein the diluent is one or more selected from the group consisting of water, ethanol and glycerol.

8. The pharmaceutical composition according to any one of claims 1-7, comprising the compound of formula (I), the surfactant, the buffering agent, the osmotic pressure regulator, the metal chelating agent and the diluent, wherein the compound of formula (I) has a concentration of 0.002 mg/mL to 50 mg/mL, the surfactant has a concentration of 0.02 mg/mL to 3 mg/mL, the buffering agent has a concentration of 0.1 mg/mL to about 25 mg/mL, the osmotic pressure regulator has a concentration of 5 mg/mL to 9 mg/mL, and the metal chelating agent has a concentration of 0.01 mg/mL to about 5 mg/mL.

9. The pharmaceutical composition according to any one of claims 1-8, comprising the compound of formula (I), Tween 80, sodium dihydrogen phosphate or monohydrate thereof, disodium hydrogen phosphate, sodium chloride, disodium edetate and water; wherein optionally, the compound of formula (I) has a concentration of 0.002 mg/mL to 50 mg/mL, Tween 80 has a concentration of 0.02 mg/mL to 3 mg/mL, sodium dihydrogen phosphate and disodium hydrogen phosphate have a concentration of 0.1 mg/mL to 25 mg/mL, sodium chloride has a concentration of 5 mg/mL to 9 mg/mL, and disodium edetate has a concentration of 0.01 mg/mL to about 5 mg/mL.

10. The pharmaceutical composition according to any one of claims 1-9, wherein the compound of formula (I) is a crystalline form of the compound of formula (I) which comprises 5, 6, 7, 8, 9, 10 or 11 diffraction peaks in an X-ray powder diffraction pattern using Cu Kα radiation selected from the following 2Θ angles: 5.81±0.2°, 8.38±0.2°, 11.16±0.2°, 13.96±0.2°, 14.47±0.2°, 15.01±0.2°, 16.76±0.2°, 17.95±0.2°, 20.83±0.2°, 24.73±0.2° and 26.13±0.2°; or, having diffraction peaks in an X-ray powder diffraction pattern using Cu Kα radiation at the following 2Θ: 5.81±0.2°, 13.96±0.2°, 15.01±0.2°, 17.95±0.2° and 24.73±0.2°; or, having diffraction peaks at the following 2Θ: 5.81±0.2°, 8.38±0.2°, 11.16±0.2°, 13.96±0.2°, 14.47±0.2°, 15.01±0.2°, 16.76±0.2°, 17.95±0.2°, 20.83±0.2°, 24.73±0.2° and 26.13±0.2°.

11. The pharmaceutical composition according to any one of claims 1-10, wherein the compound of formula (I) or the pharmaceutically acceptable salt thereof has a particle size of X₅₀ < 10 µm; or the compound of formula (I) or the pharmaceutically acceptable salt thereof has the particle size of X₅₀ ≤ 5 µm and X₉₀ < 10 µm.

12. The pharmaceutical composition according to any one of claims 1-11, wherein the mass ratio of the compound of formula (I) or the pharmaceutically acceptable salt thereof to the surfactant is about 1:200 to 100:1, preferably about 1:150 to 50:1, more preferably about 1:50 to 25:1, and even more preferably about 1:1 to 15:1, and the mass of the compound of formula (I) or the pharmaceutically acceptable salt thereof is based on the compound of formula (I).

13. The pharmaceutical composition according to any one of claims 1-12, wherein the pharmaceutical composition is in the form of a suspension.

14. A method for preparing the pharmaceutical composition according to claim 2, comprising: mixing the surfactant, the compound of formula (I) or the pharmaceutically acceptable salt thereof, and at least one selected from the group consisting of: the metal chelating agent, the buffering agent, the diluent, and the osmotic pressure regulator.

15. A pharmaceutical composition according to any one of claims 1-14 for use in preventing or treating the condition associated with PDE3 and/or PDE4; optionally, the condition associated with PDE3 and/or PDE4 is selected from the group consisting of asthma or chronic obstructive pulmonary disease.

## Patentansprüche

1. Pharmazeutische Zusammensetzung, umfassend: eine Verbindung der Formel (I) oder ein pharmazeutisch verträgliches Salz davon und ein Tensid,

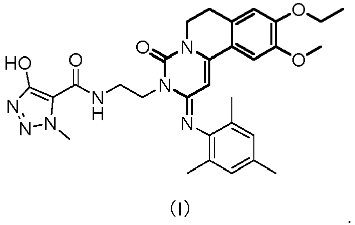

2. Pharmazeutische Zusammensetzung nach Anspruch 1, umfassend die Verbindung der Formel (I) oder das pharmazeutisch verträgliche Salz davon, das Tensid und mindestens ein Puffermittel, einen osmotischen Druckregler, einen Metall-Chelatbildner und ein Verdünnungsmittel.

3. Pharmazeutische Zusammensetzung nach Anspruch 1 oder 2, wobei das Tensid ein nichtionisches Tensid ist;
oder das Tensid eines oder mehrere ist, ausgewählt aus der Gruppe bestehend aus Polyoxyethylenglykol, Polypropylenglykolalkylether, Alkylpolyglucosid, Octylphenolpolyoxyethylenether, Alkylphenolpolyoxyethylenether, Glycerinalkylester, Polyoxyethylen-Sorbitanfettsäureester, Sorbitanalkylester, Sorbitanfettsäureester, Cocamid MEA, Cocamid DEA, Dodecyldimethylaminoxid, ein Blockcopolymer aus Polyethylenglykol und Polypropylenglykol und polyethoxyliertes Tallowamin;
oder das Tensid ist eines oder mehrere, ausgewählt aus der Gruppe, bestehend aus einem Tween und einem Span;
oder das Tensid ist eines oder mehrere, ausgewählt aus der Gruppe, bestehend aus Tween 20, Tween 80 und Span 20; oder
oder das Tensid hat eine Konzentration von etwa 0,01 mg/ml bis etwa 8 mg/ml.

4. Pharmazeutische Zusammensetzung nach Anspruch 2, wobei das Puffermittel eines oder mehrere ist, ausgewählt aus der Gruppe bestehend aus Schwefelsäure, Salzsäure, Natriumhydroxid, Zitronensäure, Natriumcitrat, Milchsäure, Natriumlactat, Essigsäure, Natriumacetat, Trinatriumphosphat, Natriumdihydrogenphosphat, Dinatriumhydrogenphosphat, Kaliumdihydrogenphosphat, Weinsäure, Natriumtartrat, Glycin, Borsäure und Phthalsäure;
oder das Puffermittel ist ausgewählt aus der Gruppe bestehend aus Zitronensäure, Citrat, Weinsäure, Tartrat, Phosphorsäure und Phosphat;
oder das Puffermittel ist ausgewählt aus der Gruppe, bestehend aus einem Zitrat, einem Tartrat und einem Phosphat;
oder das Puffermittel hat eine Konzentration von etwa 0,01 mg/ml bis etwa 50 mg/ml;
oder das Puffermittel wird verwendet, um einen pH-Wert der pharmazeutischen Zusammensetzung zwischen etwa 3,0 und etwa 8,5 zu halten.

5. Pharmazeutische Zusammensetzung nach Anspruch 2, wobei der osmotische Druckregler einer oder mehrere ist, ausgewählt aus der Gruppe, bestehend aus Natriumchlorid, Kaliumchlorid, Glucose, Mannitol und Xylitol.

6. Pharmazeutische Zusammensetzung nach Anspruch 2, wobei der Metall-Chelatbildner ein oder mehrere Mittel ist, ausgewählt aus der Gruppe, bestehend aus Edetinsäure, Dinatriumedetat und Calciumdinatriumedetat besteht.

7. Pharmazeutische Zusammensetzung nach Anspruch 2, wobei das Verdünnungsmittel eines oder mehrere aus der Gruppe bestehend aus Wasser, Ethanol und Glycerin ist.

8. Pharmazeutische Zusammensetzung nach einem der Ansprüche 1-7, umfassend die Verbindung der Formel (I), das Tensid, das Puffermittel, den osmotischen Druckregler, den Metall-Chelatbildner und das Verdünnungsmittel, wobei die Verbindung der Formel (I) eine Konzentration von 0,002 mg/ml bis 50 mg/ml hat, das Tensid eine Konzentration von 0,02 mg/ml bis 3 mg/ml hat, das Puffermittel eine Konzentration von 0,1 mg/ml bis etwa 25 mg/ml hat, der osmotische Druckregler eine Konzentration von 5 mg/ml bis 9 mg/ml hat und der Metall-Chelatbildner eine Konzentration von 0,01 mg/ml bis etwa 5 mg/ml hat.

9. Pharmazeutische Zusammensetzung nach einem der Ansprüche 1-8, umfassend die Verbindung der Formel (I), Tween 80, Natriumdihydrogenphosphat oder Monohydrat davon, Dinatriumhydrogenphosphat, Natriumchlorid, Dinatriumedetat und Wasser; wobei optional die Verbindung der Formel (I) eine Konzentration von 0,002 mg/ml bis 50 mg/ml hat, Tween 80 eine Konzentration von 0,02 mg/ml bis 3 mg/ml hat, Natriumdihydrogenphosphat und Dinatriumhydrogenphosphat eine Konzentration von 0,1 mg/ml bis 25 mg/ml haben, Natriumchlorid eine Konzentration von 5 mg/ml bis 9 mg/ml hat und Dinatriumedetat eine Konzentration von 0,01 mg/ml bis etwa 5 mg/ml hat.

10. Pharmazeutische Zusammensetzung nach einem der Ansprüche 1-9, wobei die Verbindung der Formel (I) eine kristalline Form der Verbindung der Formel (I) ist, die 5, 6, 7, 8, 9, 10 oder 11 Beugungspeaks in einem Röntgenpulverbeugungsmuster unter Verwendung von Cu Kα-Strahlung umfasst, die aus den folgenden 2θ-Winkeln ausgewählt sind: 5. 81±0,2°, 8,38±0,2°, 11,16±0,2°, 13,96±0,2°, 14,47±0,2°, 15,01±0,2°, 16,76±0,2°, 17,95±0,2°, 20,83±0,2°, 24,73±0,2° und 26,13±0,2°; oder mit Beugungspeaks in einem Röntgenpulverbeugungsmuster unter Verwendung von Cu Kα-Strahlung bei den folgenden 2Θ: 5,81±0,2°, 13,96±0,2°, 15,01±0,2°, 17,95±0,2° und 24,73±0,2°; oder mit Beugungspeaks bei den folgenden 2Θ: 5,81±0,2°, 8,38±0,2°, 11,16±0,2°, 13,96±0,2°, 14,47±0,2°, 15,01±0,2°, 16,76±0,2°, 17,95±0,2°, 20,83±0,2°, 24,73±0,2° und 26,13±0,2°.

11. Pharmazeutische Zusammensetzung nach einem der Ansprüche 1-10, wobei die Verbindung der Formel (I) oder das pharmazeutisch verträgliche Salz davon eine Teilchengröße von X₅₀ ≤ 10 µm aufweist; oder die Verbindung der Formel (I) oder das pharmazeutisch verträgliche Salz davon eine Teilchengröße von X₅₀ ≤ 5 µm und X₉₀ ≤ 10 µm aufweist.

12. Pharmazeutische Zusammensetzung nach einem der Ansprüche 1-11, wobei das Massenverhältnis der Verbindung der Formel (I) oder des pharmazeutisch verträglichen Salzes davon zu dem Tensid etwa 1:200 bis 100:1, vorzugsweise etwa 1:150 bis 50:1, weiter bevorzugt etwa 1:50 bis 25:1 und noch bevorzugter etwa 1:1 bis 15:1 beträgt und die Masse der Verbindung der Formel (I) oder des pharmazeutisch verträglichen Salzes davon auf die Verbindung der Formel (I) bezogen ist.

13. Pharmazeutische Zusammensetzung nach einem der Ansprüche 1-12, wobei die pharmazeutische Zusammensetzung in Form einer Suspension vorliegt.

14. Verfahren zur Herstellung der pharmazeutischen Zusammensetzung nach Anspruch 2, umfassend: Mischen des Tensids, der Verbindung der Formel (I) oder des pharmazeutisch verträglichen Salzes davon und mindestens eines, ausgewählt aus der Gruppe, bestehend aus: dem Metall-Chelatbildner, dem Puffermittel, dem Verdünnungsmittel und dem osmotischen Druckregler.

15. Pharmazeutische Zusammensetzung nach einem der Ansprüche 1-14 zur Verwendung bei der Vorbeugung oder Behandlung des mit PDE3 und/oder PDE4 assoziierten Zustands; optional ist der mit PDE3 und/oder PDE4 assoziierte Zustand ausgewählt aus der Gruppe bestehend aus Asthma oder chronisch obstruktiver Lungenerkrankung.

## Revendications

1. Composition pharmaceutique comprenant : un composé de formule (I) ou un sel pharmaceutiquement acceptable de celui-ci et un surfactant,

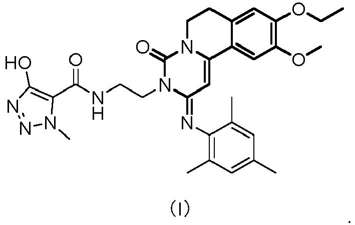

2. Composition pharmaceutique selon la revendication 1, comprenant le composé de formule (I) ou son sel pharmaceutiquement acceptable, le surfactant et au moins un agent tampon, un régulateur de pression osmotique, un agent chélateur de métaux et un diluant.

3. Composition pharmaceutique selon la revendication 1 ou 2, dans laquelle le surfactant est un surfactant non ionique ;
ou le surfactant est un ou plusieurs agents choisis dans le groupe constitué par le polyoxyéthylène glycol, l'éther d'alkyle de polypropylène glycol, le polyglucoside d'alkyle, l'éther de polyoxyéthylène d'octylphénol, l'éther de polyoxyéthylène d'alkylphénol, l'ester d'alkyle de glycérine, l'ester d'acide gras de sorbitane polyoxyéthylène, l'ester d'alkyle de sorbitane, l'ester d'acide gras de sorbitane, le cocamide MEA, le cocamide DEA, l'oxyde de dodécyldiméthylamine, un copolymère séquencé de polyéthylène glycol et de polypropylène glycol, et l'amine de suif polyéthoxylée ;
ou le surfactant est un ou plusieurs agents choisis dans le groupe constitué d'un Tween et d'un Span ;
ou le surfactant est un ou plusieurs agents choisis dans le groupe constitué par le Tween 20, le Tween 80 et le Span 20 ;
ou le surfactant a une concentration d'environ 0,01 mg/ml à environ 8 mg/ml.

4. Composition pharmaceutique selon la revendication 2, dans laquelle l'agent tampon est un ou plusieurs agents choisis dans le groupe constitué par l'acide sulfurique, l'acide chlorhydrique, l'hydroxyde de sodium, l'acide citrique, le citrate de sodium, l'acide lactique, le lactate de sodium, l'acide acétique, l'acétate de sodium, le phosphate trisodique, le dihydrogénophosphate de sodium, l'hydrogénophosphate disodique, le dihydrogénophosphate de potassium, l'acide tartrique, le tartrate de sodium, la glycine, l'acide borique et l'acide phtalique ;
ou l'agent tampon est choisi dans le groupe constitué par l'acide citrique, un citrate, l'acide tartrique, un tartrate, l'acide phosphorique et un phosphate ;
ou l'agent tampon est choisi dans le groupe constitué d'un citrate, d'un tartrate et d'un phosphate ;
ou l'agent tampon a une concentration d'environ 0,01 mg/ml à environ 50 mg/ml ;
ou l'agent tampon est utilisé pour régler le pH de la composition pharmaceutique entre environ 3,0 et environ 8,5.

5. Composition pharmaceutique selon la revendication 2, dans laquelle le régulateur de pression osmotique est un ou plusieurs agents choisis dans le groupe constitué par le chlorure de sodium, le chlorure de potassium, le glucose, le mannitol et le xylitol.

6. Composition pharmaceutique selon la revendication 2, dans laquelle l'agent chélateur de métaux est un ou plusieurs agents choisis dans le groupe constitué de l'acide édétique, de l'édétate disodique et de l'édétate disodique de calcium.

7. Composition pharmaceutique selon la revendication 2, dans laquelle le diluant est un ou plusieurs agents choisis dans le groupe constitué par l'eau, l'éthanol et le glycérol.

8. Composition pharmaceutique selon l'une quelconque des revendications 1 à 7, comprenant le composé de formule (I), le surfactant, l'agent tampon, le régulateur de pression osmotique, l'agent chélateur de métaux et le diluant, dans laquelle le composé de formule (I) a une concentration de 0,002 mg/mm à 50 mg/ml, le surfactant a une concentration de 0,02 mg/ml à 3 mg/ml, l'agent tampon a une concentration de 0,1 mg/ml à environ 25 mg/ml, le régulateur de pression osmotique a une concentration de 5 mg/ml à 9 mg/ml et l'agent chélateur de métaux a une concentration de 0,01 mg/ml à environ 5 mg/ml.

9. Composition pharmaceutique selon l'une quelconque des revendications 1 à 8, comprenant le composé de formule (I), le Tween 80, le dihydrogénophosphate de sodium ou son monohydrate, l'hydrogénophosphate disodique, le chlorure de sodium, l'édétate disodique et l'eau ; dans laquelle, en option, le composé de formule (I) a une concentration de 0,002 mg/ml à 50 mg/ml, le Tween 80 a une concentration de 0,02 mg/ml à 3 mg/ml, le dihydrogénophosphate de sodium et l'hydrogénophosphate disodique ont une concentration de 0,1 mg/ml à 25 mg/ml, le chlorure de sodium a une concentration de 5 mg/ml à 9 mg/ml et l'édétate disodique a une concentration de 0,01 mg/ml à environ 5 mg/ml.

10. Composition pharmaceutique selon l'une quelconque des revendications 1 à 9, dans laquelle le composé de formule (I) est une forme cristalline du composé de formule (I) qui comprend 5, 6, 7, 8, 9, 10 ou 11 pics de diffraction dans un diagramme de diffraction des rayons X sur poudre utilisant un rayonnement Cu Kα choisi parmi les angles 2Θ suivants : 5,81±0,2°, 8,38±0,2°, 11,16±0,2°, 13,96±0,2°, 14,47±0,2°, 15,01±0,2°, 16,76±0,2°, 17,95±0,2°, 20,83±0,2°, 24,73±0,2° et 26,13±0,2° ; ou présentant des pics de diffraction dans un diagramme de diffraction des rayons X sur poudre utilisant un rayonnement Cu Kα aux angles 2Θ suivants : 5,81±0,2°, 13,96±0,2°, 15,01±0,2°, 17,95±0,2° et 24,73±0,2° ; ou présentant des pics de diffraction aux 2Θ suivants : 5,81±0,2°, 8,38±0,2°, 11,16±0,2°, 13,96±0,2°, 14,47±0,2°, 15,01±0,2°, 16,76±0,2°, 17,95±0,2°, 20,83±0,2°, 24,73±0,2° et 26,13±0,2°.

11. Composition pharmaceutique selon l'une quelconque des revendications 1 à 10, dans laquelle le composé de formule (I) ou son sel pharmaceutiquement acceptable a une taille de particules X₅₀ ≤ 10 µm ; ou le composé de formule (I) ou son sel pharmaceutiquement acceptable a une taille de particules X₅₀ ≤ 5 µm et X₉₀ < 10 µm.

12. Composition pharmaceutique selon l'une quelconque des revendications 1 à 11, dans laquelle le rapport massique entre le composé de formule (I) ou son sel pharmaceutiquement acceptable et le surfactant est d'environ 1:200 à 100:1, de préférence d'environ 1:150 à 50:1, plus préférentiellement d'environ 1:50 à 25:1, et encore plus préférentiellement d'environ 1:1 à 15:1, et la masse du composé de formule (I) ou de son sel pharmaceutiquement acceptable est calculée sur la base du composé de formule (I).

13. Composition pharmaceutique selon l'une quelconque des revendications 1 à 12, dans laquelle la composition pharmaceutique se présente sous la forme d'une suspension.

14. Procédé de préparation de la composition pharmaceutique selon la revendication 2, consistant à : mélanger le surfactant, le composé de formule (I) ou son sel pharmaceutiquement acceptable, et au moins un agent choisi dans le groupe constitué par : l'agent chélateur de métaux, l'agent tampon, le diluant, et le régulateur de pression osmotique.

15. Composition pharmaceutique selon l'une quelconque des revendications 1 à 14 pour utilisation dans la prévention ou le traitement de l'état associé à la PDE3 et/ou à la PDE4 ; en option, l'état associé à la PDE3 et/ou à la PDE4 est choisi dans le groupe constitué par l'asthme ou la bronchopneumopathie chronique obstructive.
